# EUROPEAN PATENT SPECIFICATION

(11) **EP 4 125 606 B1**
(45) Date of publication and mention of the grant of the patent: **16.10.2024**
(21) Application number: 21713942.7
(22) Date of filing: 22.03.2021
(51) Int. Cl.: A61B 8/08

(54) **SYSTEMS AND METHODS FOR IMAGING AND MEASURING EPICARDIAL ADIPOSE TISSUE**
VORRICHTUNG UND VERFAHREN ZUR BILDGEBUNG UND MESSUNG DES EPIKARDIALEN ADIPÖSEN GEWEBES
SYSTÈME ET PROCÉDÉ POUR L'IMAGÉRIE ET LA DETERMINATION D'UN TISSU ADIPEUX ÉPICARDIQUE

(30) Priority: 27.03.2020 US 202063000820 P
(43) Date of publication of application: 08.02.2023
(73) Proprietor: Koninklijke Philips N.V., 5656 AG Eindhoven (NL)
(72) Inventor: ERRICO, Claudia, 5656 AE Eindhoven (NL); LI, Qianxi, 5656 AE Eindhoven (NL); ERKAMP, Ramon Quido, 5656 AE Eindhoven (NL); XIE, Hua, 5656 AE Eindhoven (NL)
(74) Representative: Philips Intellectual Property & Standards
(86) International application number: PCT/EP2021/057164
(87) International publication number: WO 2021/191092

(56) References cited:
- DE-A1- 102008 014 899
- US-A1- 2019 388 060
- SAURA D ET AL: "Reproducibility of echocardiographic measurements of epicardial fat thickness", INTERNATIONAL JOURNAL OF CARDIOLOGY, ELSEVIER, AMSTERDAM, NL, vol. 141, no. 3, 11 June 2010 (2010-06-11), pages 311 - 313, XP027087427, ISSN: 0167-5273, [retrieved on 20100611]

## Description

### TECHNICAL FIELD

The present disclosure pertains to imaging systems and methods for imaging and measuring epicardial adipose tissue. In particular, imaging systems and methods for imaging and measuring epicardial adipose tissue in ultrasound images.

### BACKGROUND

Coronary angiography is the preferred method to see the heart's blood vessels. It is clearly an invasive procedure, but to date it is the gold standard technique for the evaluation of coronary artery disease (CAD). In recent years, a large number of retrospective clinical studies have suggested that fat deposition in visceral organs and epicardial tissue could serve as a predictor of CAD.

The heart and the vessels are surrounded by layers of adipose tissue, whose thickness and volume can be quantified via ultrasound imaging, computed tomography (CT), or magnetic resonance imaging (MRI). Anatomically, epicardial adipose tissue (EAT) is located between the outer wall of the myocardium and the visceral layer of pericardium. Hence, for its vicinity and closeness to the myocardium, not only do they share the same microcirculation, but EAT is considered to be a component of visceral adiposity and associated with metabolic syndrome (MS). MS is an ensemble of risk factors including abdominal adiposity, impaired fasting glucose, hypertension, and dyslipidemia. The onset of MS exposes patients to cardiovascular disease and diabetes; hence, individuals with MS have an increased risk for CAD when compared to individuals without MS.

Clinical studies have demonstrated how EAT thickness can be related to presence/absence of CAD along with MS. Studies also report positive correlation between EAT thickness and the Gensini score and CAD with and/or without MS. Several clinical studies have demonstrated how EAT thickness can be related to presence/absence of CAD along with MS. A study has also demonstrated that EAT measurements are correlated to other risk factors such as obesity and HDL-C (high density lipoprotein cholesterol) as predictors of CAD. EAT thickness may also be an independent risk factor associated with CAD. Accordingly, reliable EAT measurements are desirable. DE 10 2008 014899 describes a method of determining a ratio of epicardial fat and heart tissue. US 2019/388060 describes an imaging protocol. Saura D et. Al "Reproducibility of echocardiographic measurements of epicardial fat thickness" International Journal of Cardiolohy, Elsevier, Amsterdam, NL, vol. 141, no.3, 11 June 2010, pages 311-313 describes measuring epicardial fat thickness.

### SUMMARY

The present disclosure describes systems and methods for imaging and measuring epicardial adipose tissue (EAT). In some examples, a deep learning enabled ultrasound imaging screening tool may enhance EAT visualization and provide robust thickness measurement in an optimal cardiac view. An ultrasound imaging system may identifying a proper cardiac view by recognizing anatomical cardiac landmarks in an ultrasonic image, activating an EAT imaging mode and adjust imaging frequency, depth of focus, and/or gain, dynamic range, and time gain compensation of the beam to improve visualization of the EAT, segment and contour the EAT image feature, and/or perform dynamic measurement of the EAT thickness during various cardiac phases such as diastole and systole. Optionally, in some embodiments, which do not form part of the claimed invention, the ultrasound imaging system may determine a coronary artery disease (CAD) assessment using patient information from a patient's medical history (e.g., medical records) input with the EAT thickness.

An ultrasound imaging system according to an example of the present disclosure may include a processor configured to analyze a first ultrasound image to determine whether a proper cardiac view has been acquired, when the proper cardiac view has been acquired, adjust imaging parameters, cause an ultrasound probe to acquire a second ultrasound image with the adjusted imaging parameters, and segment epicardial adipose tissue (EAT) from the second ultrasound image to generate a segmented image.

A method according to an example of the present disclosure may include determining whether a proper cardiac view has been acquired in a first ultrasound image, responsive to determining the proper cardiac view has been acquired, generating adjusted imaging parameters, acquiring a second ultrasound image with the adjusted imaging parameters, segmenting epicardial adipose tissue (EAT) from the second ultrasound image to generate a segmented image, and acquiring measurements of the EAT from the segmented image.

In accordance with an example of the present disclosure, a non-transitory computer-readable medium may contain instructions, that when executed, may cause an imaging system to determine whether a proper cardiac view has been acquired in a first ultrasound image, responsive to determining the proper cardiac view has been acquired, generate adjusted imaging parameters, acquire a second ultrasound image with the adjusted imaging parameters, segment epicardial adipose tissue (EAT) from the second ultrasound image to generate a segmented image, and acquire measurements of the EAT from the segmented image.

### BRIEF DESCRIPTION OF THE DRAWINGS

**FIG. 1** is a block diagram of an ultrasound system in accordance with principles of the present disclosure.
**FIG. 2** is a block diagram illustrating an example processor in accordance with principles of the present disclosure.
**FIG. 3** is a block diagram of a process for training and deployment of a neural network in accordance with the principles of the present disclosure.
**FIG. 4** shows a process for training and deployment of a neural network in accordance with the principles of the present disclosure.
**FIG. 5** shows an example of a neural network in accordance with the principles of the present disclosure.
**FIG. 6A** is an example of a dual-display of ultrasound images in accordance with the principles of the present disclosure.
**FIG. 6B** is an example of a display of an ultrasound image in accordance with the principles of the present disclosure.
**FIG. 7** is an example of a patient dashboard in accordance with the principles of the present disclosure.
**FIG. 8** is a flow chart of a method in accordance with the principles of the present disclosure.

### DETAILED DESCRIPTION

The following description of certain embodiments is merely exemplary in nature and is in no way intended to limit the invention or its applications or uses. In the following detailed description of embodiments of the present systems and methods, reference is made to the accompanying drawings which form a part hereof, and which are shown by way of illustration specific embodiments in which the described systems and methods may be practiced. These embodiments are described in sufficient detail to enable those skilled in the art to practice presently disclosed systems and methods, and it is to be understood that other embodiments may be utilized and that structural and logical changes may be made without departing from the scope of the appended claims. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

Coronary angiography is usually performed to evaluate the severity of coronary arteriosclerotic lesions. However, this is a very invasive procedure. As noted previously, clinical studies have revealed the association of epicardial adipose tissue (EAT) thickness with severity of coronary artery disease (CAD). As an alternative to angiography, the EAT may be visualized by ultrasound imaging. The EAT is usually a feature recognizable on top of the right ventricle while cardiac chambers and main vessels may be located toward the center and bottom of the ultrasound images. However, performing transthoracic ultrasound requires high skillsets, and as a result, manual measurements of EAT done on the ultrasound images may be operator dependent.

In current clinical practice, EAT measurements are not yet taken into consideration due to the high skillset required to identify the layer of adipose tissue on top of the right ventricle in the parasternal (PLAX) long view and short-axis (PSAX) view. Vertical and/or horizontal EAT thickness measurements are manually acquired by the user in these views. Thus, variations in the imaging planes and measurement acquisition technique may lead to irreproducible EAT measurements. Furthermore, cardiac phased array transducers have a central frequency around 2.5MHz and a depth focus around 10+ cm, which is deeper than the location of EAT (e.g., ~2-5 cm). Thus, the default cardiac acoustic imaging pre-sets (e.g., imaging parameters) may not be optimal to image the layer of adipose tissue located in the near field. A consistent image plane orientation, improved imaging parameters, and/or automatic segmenting of the EAT from images may improve reliability of EAT measurements.

Metabolic syndrome (MS) is usually related to a series of risk factors occurring all at the same time (e.g., high BMI, large waist circumference, diabetes, smoking, HDL-C, and other risk factors). Clinical studies have correlated CAD to EAT thickness and other presence of risk factors. Accordingly, may be desirable to report to a physician or other healthcare worker whether more in-depth follow-up appointments are required to rule out the presence/absence, or onset of CAD.

Disclosed herein are deep learning enabled systems and methods for enhanced EAT visualization with ultrasound imaging. The systems and methods may provide robust thickness measurement in an optimal cardiac view. Optionally, if a patient's medical history is available, a dashboard where the patient's information and EAT thickness measurement may be combined to generate a report that may suggest follow-up appointments to determine if the patient has CAD. The patient journey in CAD assessment usually starts with collecting patient history and then scheduling a chest X-ray. Doctors may also want to perform stress test, angiography, and the evaluation of coronary artery calcium scoring (CAC) via computed tomography (CT). Identifying and suggesting the appropriate follow-up procedure in the report may provide a non-invasive screening tool for CAD assessment via sonographic EAT thickness measurements.

According to examples of the present disclosure, one or more deep learning networks may be trained and implemented to identify a proper cardiac view. A proper cardiac view may be a particular standard view. As is well known in ultrasound imaging, various exams, such as echo cardiology exams, acquire a set of standard views of the organ (e.g., heart, liver) being imaged. Standard views are defined by one or more criteria such as the acoustic window from which the view is acquired, the position of the patient during acquisition, and/or what anatomical features are visible in the image. Examples of standard views in a cardiac exam include PLAX, PSAX, and subxiphoid views. In some examples, the proper cardiac views for visualizing and obtaining measurements of EAT may be the PLAX view and/or PSAX view. In some examples, the proper cardiac views may be identified by recognizing anatomical cardiac landmarks (e.g., epicardial fat, cardiac chambers, cardiac valves, aorta, etc.).

In some examples, once a proper cardiac view is identified (e.g., based on the position of anatomical landmarks within the ultrasound image), an EAT imaging mode may be activated. The acoustic settings may be different acoustic settings from the ones used for typical parasternal views. For example, the EAT imaging mode may select a higher imaging frequency (e.g., approximately 5 MHz) within the transducer bandwidth. The higher frequency may provide better near field spatial resolution to enhance image quality for EAT. The field of view may be shallower However, the image depth for EAT enhancement is approximately 5cm, which is shallower than typical field of view in cardiac imaging. Alternatively or additionally, the depth of focus may be adjusted based on the location of the EAT. In some examples, in the near-field, gain, dynamic range and/or time gain compensation (TCG) may be adjusted. In some examples, the settings may be based on the position of the EAT and/or anatomical cardiac landmarks visible on the ultrasound image. In some examples, the ultrasound imaging system may steer the ultrasound beam for a better visualization of the EAT based on the position of the EAT and/or other anatomical cardiac landmarks.

In some examples, once an image of the EAT in a proper cardiac view is acquired, the EAT may be identified and segmented and contoured from the image. Measurements (e.g., thickness across the long and short axes) may be acquired from the segmented and contoured EAT. In some examples, dynamic measurements may be acquired to capture the thickness of the EAT during different points in the cardiac cycle (e.g., diastole and systole). In some examples, measurements acquired during the cardiac cycle according to the view (e.g., PLAX, PSAX), and/or the orientation of the measurement (e.g., measurements are taken vertically or horizontally) may be tabulated and stored.

Optionally, in some examples, if patient history is available and the EAT measurement is enabled a dashboard with patient history and EAT values for predictive analysis may be generated. In some examples, the EAT thickness measurements may be flagged if the measurements fall outside a normal range. If flagged, a more in-depth follow-up appointment to verify potential onset/presence/absence of CAD may be suggested. In some examples, the patient history and EAT measurements may be analyzed on an ultrasound imaging system. In other examples, the EAT measurements may be provided from an ultrasound imaging system to a separate computing device that has access to the patient history, and the separate computing system performs the predictive analysis.

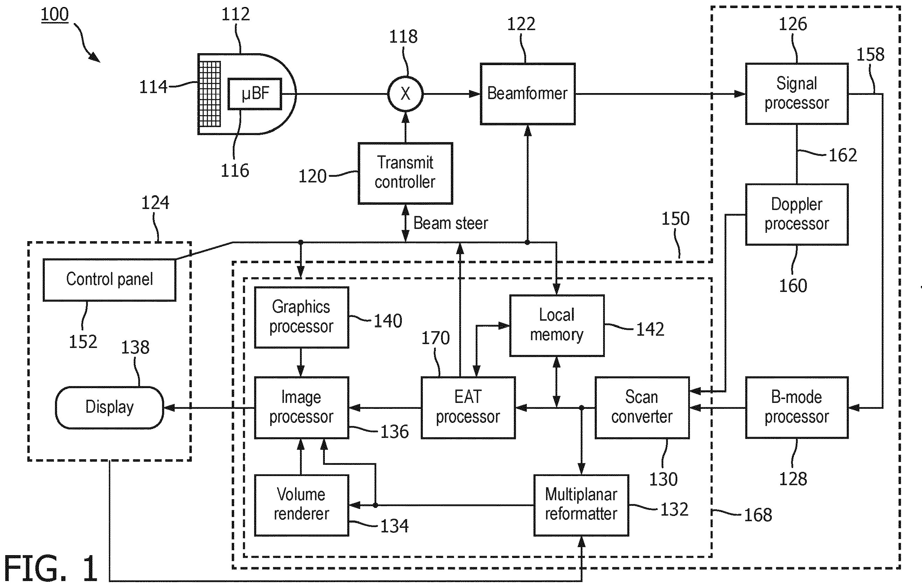

**FIG. 1** shows a block diagram of an ultrasound imaging system 100 constructed in accordance with the principles of the present disclosure. An ultrasound imaging system 100 according to the present disclosure may include a transducer array 114, which may be included in an ultrasound probe 112, for example an external probe or an internal probe such as an Intra Cardiac Echography (ICE) probe or a Trans Esophagus Echography (TEE) probe. In other embodiments, the transducer array 114 may be in the form of a flexible array configured to be conformably applied to a surface of subject to be imaged (e.g., patient). The transducer array 114 is configured to transmit ultrasound signals (e.g., beams, waves) and receive echoes responsive to the ultrasound signals. A variety of transducer arrays may be used, e.g., linear arrays, curved arrays, or phased arrays. The transducer array 114, for example, can include a two dimensional array (as shown) of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some embodiments, the transducer array 114 may be coupled to a microbeamformer 116, which may be located in the ultrasound probe 112, and which may control the transmission and reception of signals by the transducer elements in the array 114. In some embodiments, the microbeamformer 116 may control the transmission and reception of signals by active elements in the array 114 (e.g., an active subset of elements of the array that define the active aperture at any given time).

In some embodiments, the microbeamformer 116 may be coupled, e.g., by a probe cable or wirelessly, to a transmit/receive (T/R) switch 118, which switches between transmission and reception and protects the main beamformer 122 from high energy transmit signals. In some embodiments, for example in portable ultrasound systems, the T/R switch 118 and other elements in the system can be included in the ultrasound probe 112 rather than in the ultrasound system base, which may house the image processing electronics. An ultrasound system base typically includes software and hardware components including circuitry for signal processing and image data generation as well as executable instructions for providing a user interface (e.g., processing circuitry 150 and user interface 124).

The transmission of ultrasonic signals from the transducer array 114 under control of the microbeamformer 116 is directed by the transmit controller 120, which may be coupled to the T/R switch 118 and a main beamformer 122. The transmit controller 120 may control the direction in which beams are steered. Beams may be steered straight ahead from (orthogonal to) the transducer array 114, or at different angles for a wider field of view. The transmit controller 120 may also be coupled to a user interface 124 and receive input from the user's operation of a user control. The user interface 124 may include one or more input devices such as a control panel 152, which may include one or more mechanical controls (e.g., buttons, encoders, etc.), touch sensitive controls (e.g., a trackpad, a touchscreen, or the like), and/or other known input devices.

In some embodiments, the partially beamformed signals produced by the microbeamformer 116 may be coupled to a main beamformer 122 where partially beamformed signals from individual patches of transducer elements may be combined into a fully beamformed signal. In some embodiments, microbeamformer 116 is omitted, and the transducer array 114 is under the control of the main beamformer 122 which performs all beamforming of signals. In embodiments with and without the microbeamformer 116, the beamformed signals of the main beamformer 122 are coupled to processing circuitry 150, which may include one or more processors (e.g., a signal processor 126, a B-mode processor 128, a Doppler processor 160, and one or more image generation and processing components 168) configured to produce an ultrasound image from the beamformed signals (e.g., beamformed RF data).

The signal processor 126 may be configured to process the received beamformed RF data in various ways, such as bandpass filtering, decimation, I and Q component separation, and harmonic signal separation. The signal processor 126 may also perform additional signal enhancement such as speckle reduction, signal compounding, and noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, Doppler image data). For example, the system may include a B-mode signal path 158 which couples the signals from the signal processor 126 to a B-mode processor 128 for producing B-mode image data.

The B-mode processor can employ amplitude detection for the imaging of structures in the body. The signals produced by the B-mode processor 128 may be coupled to a scan converter 130 and/or a multiplanar reformatter 132. The scan converter 130 may be configured to arrange the echo signals from the spatial relationship in which they were received to a desired image format. For instance, the scan converter 130 may arrange the echo signal into a two dimensional (2D) sector-shaped format, or a pyramidal or otherwise shaped three dimensional (3D) format. The multiplanar reformatter 132 can convert echoes which are received from points in a common plane in a volumetric region of the body into an ultrasonic image (e.g., a B-mode image) of that plane, for example as described in U.S. Pat. No. 6,443,896 (Detmer). The scan converter 130 and multiplanar reformatter 132 may be implemented as one or more processors in some embodiments.

A volume renderer 134 may generate an image (also referred to as a projection, render, or rendering) of the 3D dataset as viewed from a given reference point, e.g., as described in U.S. Pat. No. 6,530,885 (Entrekin et al.). The volume renderer 134 may be implemented as one or more processors in some embodiments. The volume renderer 134 may generate a render, such as a positive render or a negative render, by any known or future known technique such as surface rendering and maximum intensity rendering.

In some embodiments, the system may include a Doppler signal path 162 which couples the output from the signal processor 126 to a Doppler processor 160. The Doppler processor 160 may be configured to estimate the Doppler shift and generate Doppler image data. The Doppler image data may include color data which is then overlaid with B-mode (i.e. grayscale) image data for display. The Doppler processor 160 may be configured to filter out unwanted signals (i.e., noise or clutter associated with non-moving tissue), for example using a wall filter. The Doppler processor 160 may be further configured to estimate velocity and power in accordance with known techniques. For example, the Doppler processor may include a Doppler estimator such as an auto-correlator, in which velocity (Doppler frequency) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators. In some embodiments, the velocity and power estimates may undergo further threshold detection to further reduce noise, as well as segmentation and post-processing such as filling and smoothing. The velocity and power estimates may then be mapped to a desired range of display colors in accordance with a color map. The color data, also referred to as Doppler image data, may then be coupled to the scan converter 130, where the Doppler image data may be converted to the desired image format and overlaid on the B-mode image of the tissue structure to form a color Doppler or a power Doppler image.

According to principles of the present disclosure, output from the scan converter 130, such as B-mode images and/or Doppler images, referred to collectively as ultrasound images, may be provided to an EAT processor 170. The EAT processor 170 may analyze the ultrasound images to determine whether the ultrasound image is of a proper cardiac view, such as the PLAX or SLAX view. In some examples, whether the ultrasound image is of a proper cardiac view is based, at least in part, on a classification of anatomical cardiac landmarks (e.g., ventricles, aortic root, mitral valve). In some examples, the EAT processor 170 may initially perform landmark detection and output the position of predicted bounding boxes (BB) in image coordinates (xo, zo, width of the BB, height of BB). Once the proper cardiac view has been identified, the EAT processor 170 may adjust acoustic and/or other imaging parameters (collectively imaging parameters) of the imaging system 100 to improve EAT visualization. For example, imaging frequency may be increased, depth of focus may be decreased, and/or dynamic range may be adjusted to improve EAT visualization.

In some examples, EAT processor 170 may adjust the imaging parameters for the entire ultrasound image for enhancing the EAT visualization and cause the imaging system 100 to acquire images in an interleaved acoustic sequences. In other words, the imaging parameters will switch between settings for acquiring a standard cardiac image and settings for acquiring enhanced visualization of EAT for alternating image acquisitions (e.g., frames). In these examples, the user may see a split window with a side-by-side display of conventional parasternal (e.g., PLAX, PSAX) and an enhanced view of the EAT of top part of the right ventricle. Although the overall frame rate may be decreased by a factor of < 2X, IQ of the parasternal view IQ may be maintained whereas the EAT view may have a high spatial resolution which may improve EAT border visualization. This may permit more accurate thickness measurements of the EAT. With high spatial resolution and reduced imaging depth, may be easier to differentiate between pericardial adipose tissue and EAT. In some applications, simultaneously displaying the full parasternal view with full coverage with the enhanced EAT image may assist the user in maintaining the position of the probe and avoid deviating from this preferred view for EAT measurements.

In other examples, only the near field portion of the parasternal view is imaged. This may maintain a high resolution of all structures from chest wall to the EAT to the top part of the right ventricle. In this case since the adjustment of the imaging parameters for visualizing the EAT happens at shallow depth (near field), the imaging sequence may have an increased spatial resolution as well as a higher temporal resolution for the near field cardiac structures.

In some examples, a user may select via user interface 124 whether only the near field portion of the parasternal view is imaged or whether interleaved acoustic sequence described above is utilized. In some examples, the imaging system 100 may automatically switch to the adjusted imaging parameters once the EAT processor 170 has recognized the proper cardiac view. In other examples, the user may be prompted (e.g., audio, text and/or graphic on display 138) to switch to the adjusted imaging parameters for visualizing EAT.

Once images of the EAT with the adjusted imaging parameters are acquired, the EAT processor 170 may identify and segment the EAT from the images. In some examples, the EAT processor 170 may differentiate between the EAT and pericardial adipose tissue (PAT), which may have no correlation to CAD and/or EAT. Measurements of the EAT (e.g., thickness measurements) may be acquired from the segmented images. In some examples, the EAT processor 170 may make the measurements. In some examples, a user may make measurements on the EAT in the segmented images.

Optionally, in some examples, the EAT processor 170 may receive patient information (e.g., cholesterol levels, body max index, gender, age). The information may be retrieved from local memory 142 and/or may be provided by a user via user interface 124. The EAT processor 170 may analyze the patient information and the acquired EAT measurements to generate a report. The report may be provided as a "dashboard" on display 138 in some examples. The report may provide at least some of the patient information, EAT measurements, ultrasound image, and/or a suggestion for follow-ups (e.g., stress test, angiography). As mentioned previously, in some examples, the EAT measurements may be provided to another computing device with access to the patient information. The computing device may include a processor configured to perform the analysis of the patient information and the acquired EAT measurements to generate the report. For example, a workstation used by radiologists or other users may perform the analysis.

In some examples, images of the EAT may be acquired and segmented from one or more phases of the cardiac cycle. Measurements may be made from the images from the different phases. For example, the thickness of the EAT may be measured at systole and/or diastole. In some examples, the EAT processor 170 may identify the phase of the cardiac cycle based, at least in part, on the anatomical cardiac landmarks. In other examples, ECG triggering and/or other known methods may be used to identify the phase of the cardiac cycle in which an image was acquired.

In some embodiments, the EAT processor 170 may be implemented by one or more processors and/or application specific integrated circuits. In some embodiments, the EAT processor 170 may include any one or more machine learning, artificial intelligence algorithms, and/or multiple neural networks. In some examples, EAT processor 170 may include a deep neural network (DNN), a convolutional neural network (CNN), a recurrent neural network (RNN), an autoencoder neural network, or the like, to recognize a proper cardiac view, adjust imaging parameters, and/or identify and segment out the EAT from the view. In some embodiments, the neural network may perform multi-task learning. Examples of neural networks that may be used include, but are not limited to, Simultaneous detection and segmentation (SDS), YOLOv2, MobileNet SSD lite, SSD: Single Shot MultiBox Detector networks. The neural network may be implemented in hardware (e.g., neurons are represented by physical components) and/or software (e.g., neurons and pathways implemented in a software application) components. The neural network implemented according to the present disclosure may use a variety of topologies and learning algorithms for training the neural network to produce the desired output. For example, a software-based neural network may be implemented using a processor (e.g., single or multi-core CPU, a single GPU or GPU cluster, or multiple processors arranged for parallel-processing) configured to execute instructions, which may be stored in computer readable medium, and which when executed cause the processor to perform a trained algorithm for recognizing a proper cardiac view, adjusting imaging parameters, and/or segmenting out the EAT from the view.

In various embodiments, the neural network(s) may be trained using any of a variety of currently known or later developed learning techniques to obtain a neural network (e.g., a trained algorithm or hardware-based system of nodes) that is configured to analyze input data in the form of ultrasound images, measurements, and/or statistics. In some embodiments, the neural network may be statically trained. That is, the neural network may be trained with a data set and deployed on the EAT processor 170. In some embodiments, the neural network may be dynamically trained. In these embodiments, the neural network may be trained with an initial data set and deployed on the EAT processor 170. However, the neural network may continue to train and be modified based on ultrasound images acquired by the system 100 after deployment of the neural network on the EAT processor 170.

In some embodiments, the EAT processor 170 may not include a neural network and may instead implement other image processing techniques for object identification such as image segmentation, histogram analysis, edge detection or other shape or object recognition techniques. In some embodiments, the EAT processor 170 may implement a neural network in combination with other image processing methods.

Output (e.g., B-mode images, Doppler images) from the EAT processor 170, the scan converter 130, the multiplanar reformatter 132, and/or the volume renderer 134 may be coupled to an image processor 136 for further enhancement, buffering and temporary storage before being displayed on an image display 138. For example, in some embodiments, the image processor 136 may receive the output of the EAT processor 170 that identifies the pixels and/or voxels including EAT. The image processor 136 may overlay the identified pixels and/or voxels onto the original ultrasound image. In some embodiments, the pixels and/or voxels provided by the EAT processor 170 may be overlaid in a different color (e.g., green, red, yellow) or intensity (e.g., maximum intensity) than the pixels and/or voxels of the original ultrasound image.

Although output from the scan converter 130 is shown as provided to the image processor 136 via the EAT processor 170, in some embodiments, the output of the scan converter 130 may be provided directly to the image processor 136. A graphics processor 140 may generate graphic overlays for display with the images. These graphic overlays can contain, e.g., standard identifying information such as patient name, date and time of the image, imaging parameters, and the like. For these purposes the graphics processor may be configured to receive input from the user interface 124, such as a typed patient name or other annotations. The user interface 124 can also be coupled to the multiplanar reformatter 132 for selection and control of a display of multiple multiplanar reformatted (MPR) images.

The system 100 may include local memory 142. Local memory 142 may be implemented as any suitable non-transitory computer readable medium (e.g., flash drive, disk drive). Local memory 142 may store data generated by the system 100 including ultrasound images, executable instructions, imaging parameters, training data sets, patient information, EAT measurements, or any other information necessary for the operation of the system 100.

As mentioned previously system 100 includes user interface 124. User interface 124 may include display 138 and control panel 152. The display 138 may include a display device implemented using a variety of known display technologies, such as LCD, LED, OLED, or plasma display technology. In some embodiments, display 138 may comprise multiple displays. The control panel 152 may be configured to receive user inputs (e.g., exam type, EAT measurements). The control panel 152 may include one or more hard controls (e.g., buttons, knobs, dials, encoders, mouse, trackball or others). In some embodiments, the control panel 152 may additionally or alternatively include soft controls (e.g., GUI control elements or simply, GUI controls) provided on a touch sensitive display. In some embodiments, display 138 may be a touch sensitive display that includes one or more soft controls of the control panel 152.

In some embodiments, various components shown in FIG. 1 may be combined. For instance, image processor 136 and graphics processor 140 may be implemented as a single processor. In some embodiments, various components shown in FIG. 1 may be implemented as separate components. For example, signal processor 126 may be implemented as separate signal processors for each imaging mode (e.g., B-mode, Doppler). In some embodiments, one or more of the various processors shown in FIG. 1 may be implemented by general purpose processors and/or microprocessors configured to perform the specified tasks. In some embodiments, one or more of the various processors may be implemented as application specific circuits. In some embodiments, one or more of the various processors (e.g., image processor 136) may be implemented with one or more graphical processing units (GPU).

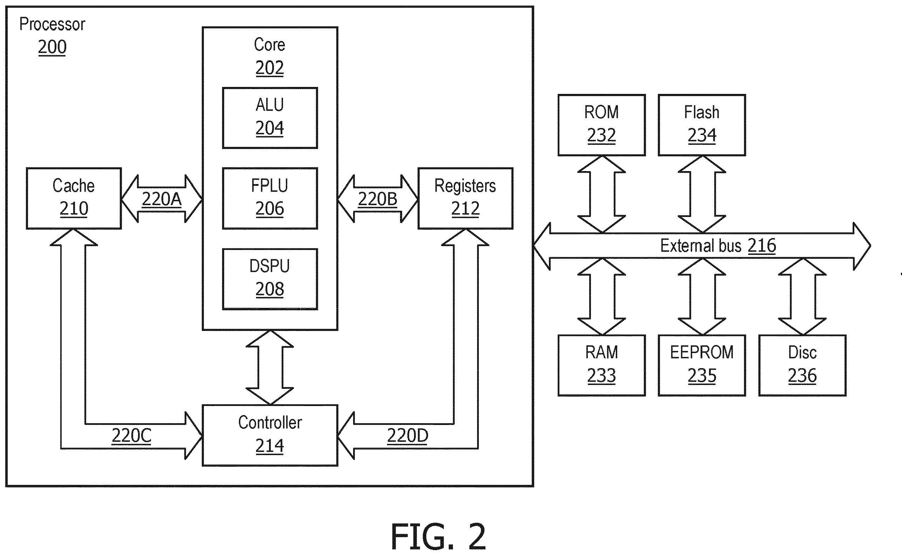

**FIG. 2** is a block diagram illustrating an example processor 200 according to principles of the present disclosure. Processor 200 may be used to implement one or more processors and/or controllers described herein, for example, image processor 136 shown in FIG. 1 and/or any other processor or controller shown in FIG. 1. Processor 200 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 200 may include one or more cores 202. The core 202 may include one or more arithmetic logic units (ALU) 204. In some embodiments, the core 202 may include a floating point logic unit (FPLU) 206 and/or a digital signal processing unit (DSPU) 208 in addition to or instead of the ALU 204.

The processor 200 may include one or more registers 212 communicatively coupled to the core 202. The registers 212 may be implemented using dedicated logic gate circuits (e.g., flip-flops) and/or any memory technology. In some embodiments the registers 212 may be implemented using static memory. The register may provide data, instructions and addresses to the core 202.

In some embodiments, processor 200 may include one or more levels of cache memory 210 communicatively coupled to the core 202. The cache memory 210 may provide computer-readable instructions to the core 202 for execution. The cache memory 210 may provide data for processing by the core 202. In some embodiments, the computer-readable instructions may have been provided to the cache memory 210 by a local memory, for example, local memory attached to the external bus 216. The cache memory 210 may be implemented with any suitable cache memory type, for example, metal-oxide semiconductor (MOS) memory such as static random access memory (SRAM), dynamic random access memory (DRAM), and/or any other suitable memory technology.

The processor 200 may include a controller 214, which may control input to the processor 200 from other processors and/or components included in a system (e.g., control panel 152 and scan converter 130 shown in FIG. 1) and/or outputs from the processor 200 to other processors and/or components included in the system (e.g., display 138 and volume renderer 134 shown in FIG. 1). Controller 214 may control the data paths in the ALU 204, FPLU 206 and/or DSPU 208. Controller 214 may be implemented as one or more state machines, data paths and/or dedicated control logic. The gates of controller 214 may be implemented as standalone gates, FPGA, ASIC or any other suitable technology.

The registers 212 and the cache 210 may communicate with controller 214 and core 202 via internal connections 220A, 220B, 220C and 220D. Internal connections may implemented as a bus, multiplexor, crossbar switch, and/or any other suitable connection technology.

Inputs and outputs for the processor 200 may be provided via a bus 216, which may include one or more conductive lines. The bus 216 may be communicatively coupled to one or more components of processor 200, for example the controller 214, cache 210, and/or register 212. The bus 216 may be coupled to one or more components of the system, such as display 138 and control panel 152 mentioned previously.

The bus 216 may be coupled to one or more external memories. The external memories may include Read Only Memory (ROM) 232. ROM 232 may be a masked ROM, Electronically Programmable Read Only Memory (EPROM) or any other suitable technology. The external memory may include Random Access Memory (RAM) 233. RAM 233 may be a static RAM, battery backed up static RAM, Dynamic RAM (DRAM) or any other suitable technology. The external memory may include Electrically Erasable Programmable Read Only Memory (EEPROM) 235. The external memory may include Flash memory 234. The external memory may include a magnetic storage device such as disc 236. In some embodiments, the external memories may be included in a system, such as ultrasound imaging system 100 shown in Fig. 1, for example local memory 142.

In some embodiments, the system 100 can be configured to implement one or more neural networks included in the EAT processor 170, which may include a CNN, to identify a cardiac view (e.g., by classifying anatomical cardiac landmarks), adjust imaging parameters, segment EAT from an ultrasound image, acquiring EAT measurements, and/or generating a suggested follow-up. The neural networks may be trained with imaging data such as image frames where one or more items of interest are labeled as present. Neural network may be trained to recognize target anatomical landmarks associated with specific standard cardiac views or a user may train neural network to locate one or more custom target anatomical features (e.g., implanted device, catheter).

In some embodiments, a neural network training algorithm associated with the neural network can be presented with thousands or even millions of training data sets in order to train the neural network to determine a confidence level for each measurement acquired from a particular ultrasound image. In various embodiments, the number of ultrasound images used to train the neural network(s) may range from about 50,000 or less to 200,000 or more. The number of images used to train the network(s) may be increased if higher numbers of different cardiac landmarks are to be identified, or to accommodate a greater variety of patient variation, e.g., weight, height, age, etc. The number of training images may differ for different items of interest or features thereof, and may depend on variability in the appearance of certain features. Training the network(s) to assess the presence of items of interest associated with features for which population-wide variability is high may necessitate a greater volume of training images.

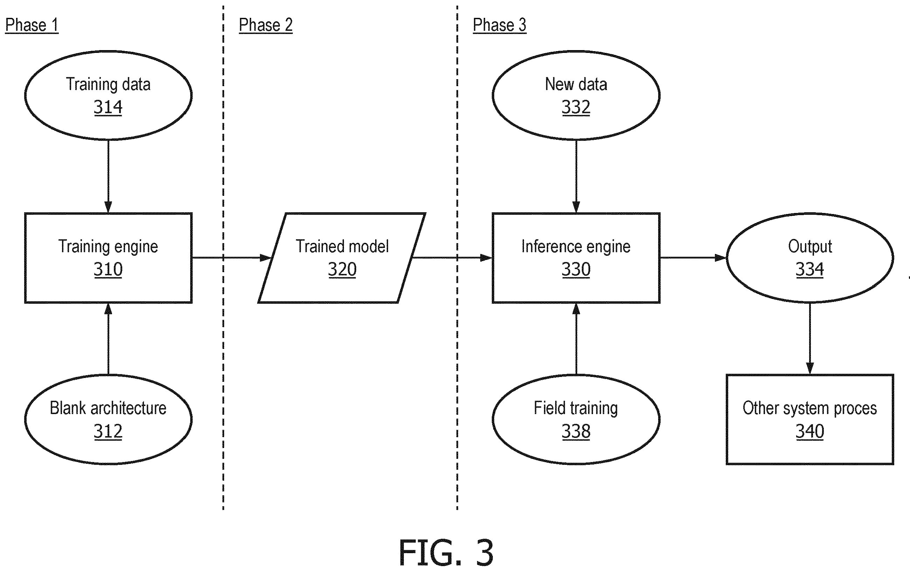

**FIG. 3** shows a block diagram of a process for training and deployment of a neural network in accordance with the principles of the present disclosure. The process shown in FIG. 3 may be used to train a neural network included in the EAT processor 170. The left hand side of FIG. 3, phase 1, illustrates the training of a neural network. To train the neural network, training sets which include multiple instances of input arrays and output classifications may be presented to the training algorithm(s) of the neural network(s) (e.g., AlexNet training algorithm, as described by Krizhevsky, A., Sutskever, I. and Hinton, *G. E.* "*ImageNet Classification with Deep Convolutional Neural Networks,"* NIPS 2012 or its descendants). Training may involve the selection of a starting network architecture 312 and the preparation of training data 314. The starting network architecture 312 may be a blank architecture (e.g., an architecture with defined layers and arrangement of nodes but without any previously trained weights) or a partially trained network, such as the inception networks, which may then be further tailored for classification of ultrasound images. The starting architecture 312 (e.g., blank weights) and training data 314 are provided to a training engine 310 for training the model. Upon sufficient number of iterations (e.g., when the model performs consistently within an acceptable error), the model 320 is said to be trained and ready for deployment, which is illustrated in the middle of FIG. 3, phase 2. The right hand side of FIG. **3****,** or phase 3, the trained model 320 is applied (via inference engine 330) for analysis of new data 332, which is data that has not been presented to the model during the initial training (in phase 1). For example, the new data 332 may include unknown images such as live ultrasound images acquired during a scan of a patient (e.g., cardiac images during an echocardiography exam). The trained model 320 implemented via engine 330 is used to classify the unknown images in accordance with the training of the model 320 to provide an output 334 (e.g., whether or not the image includes a proper cardiac view, identification of cardiac cycle phase). The output 334 may then be used by the system for subsequent processes 340 (e.g., adjust imaging parameters, identify and segment EAT from an image, acquire EAT measurements from a segmented image).

In the embodiments where the trained model 320 is used to implement a neural network of the EAT processor 170, the starting architecture may be that of a convolutional neural network, or a deep convolutional neural network, which may be trained to perform image frame indexing, image segmentation, image comparison, or any combinations thereof. With the increasing volume of stored medical image data, the availability of high-quality clinical images is increasing, which may be leveraged to train a neural network to learn the probability of a given pixel or voxel includes EAT and/or an image includes a proper cardiac view. The training data 314 may include multiple (hundreds, often thousands or even more) annotated/labeled images, also referred to as training images. It will be understood that the training image need not include a full image produced by an imagining system (e.g., representative of the full field of view of an ultrasound probe or entire MRI volume) but may include patches or portions of images of the labeled anatomical cardiac landmarks and/or EAT.

In various embodiments, the trained neural network may be implemented, at least in part, in a computer-readable medium comprising executable instructions executed by a processor, e.g., EAT processor 170.

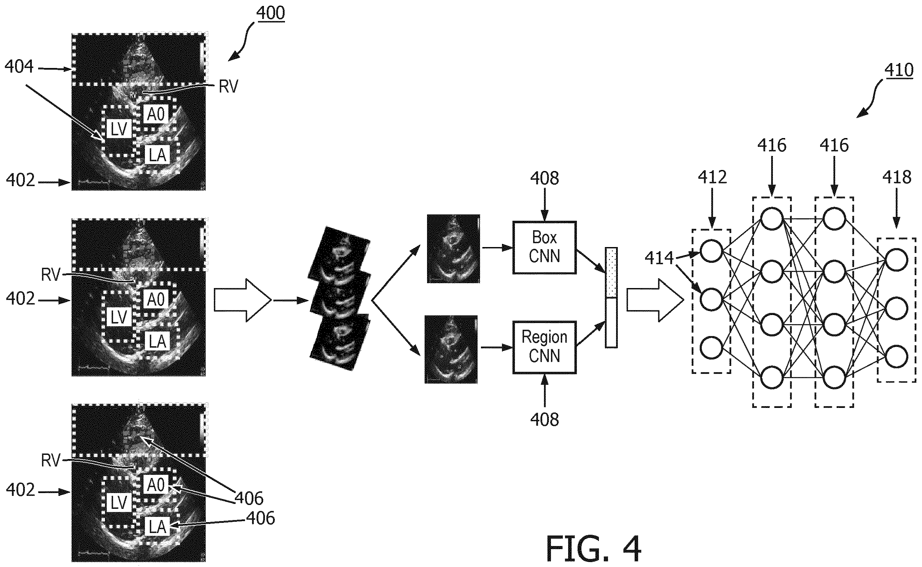

**FIG. 4** shows a process for training and deployment of a neural network in accordance with the principles of the present disclosure. In some examples, to generate a training data set 400 (e.g., ground truth images), sonographers and/or other clinicians may annotate ultrasound images 402 by placing bounding boxes 404 and/or segmentation masks (e.g., binary segmentation masks) around one or more cardiac landmarks 406 in the proper cardiac views (e.g., PLAX/PSAX views). The PLAX/PSAX may be information-rich views with a large number of cardiac landmarks 406 (e.g., right ventricle, left ventricle, mitral valve, left atrium, descending aorta, aortic valve, aortic root, pericardium, right ventricular inflow and outflow tracts). The imaging parameters used to acquire improved visualization of the EAT in the training image may also be provided. Thus, the inputs to train the deep learning networks may include ultrasound images 402 whose labels are ground truth position (or image coordinates (x, y, w, h) or segmentation masks) of cardiac landmarks 406 on the parasternal long and short axis views (e.g., bounding boxes 404 for aorta, left ventricle, left atrium, right ventricle, and EAT) along with imaging parameters. For example, an ultrasound image 402 where the aorta is located at (xA, yA, wA, hA), the EAT may have image coordinates of (xEAT, yEAT, wEAT, hEAT), and right ventricle's location may be (xRV, yRV, wRV, hRV), with the best combination of acoustic settings to visualize EAT for this configuration being: imaging frequency f = 3MHz, focus depth = 10 cm, and Gain = 60%. In some examples, inputs may include measurements of the EAT (e.g., thickness along one or more axes). In some examples, the phase of the cardiac cycle when the training image was acquired may also be provided as an input. In some examples, the neural network may receive EAT-enhanced images, whose thickness vary across the cardiac cycle, and the output may be a mask of the EAT as the heart beats and/or thickness measurements during the cardiac cycle.

The training data set 400 may be used to train one or more neural networks 408. The resulting trained neural network(s) 410 may include one or more nodes 414 in an input layer 412. The inputs may be provided to one or more hidden layers 416, which may or may not be fully connected layers. The hidden layers 416 may perform various computations on the inputs based, at least in part, on the training. The trained neural network 410 may provide one or more outputs at an output layer 418. In other examples, the neural network(s) 410 may have a different architecture than what is shown in FIG. 4.

Based on the training data set, the neural network(s) may be trained to recognize a proper cardiac view, adjust imaging parameters to visualize EAT, identify and segment EAT from images acquired with the adjusted imaging parameters, and/or make measurements on the EAT from the segmented images. In some embodiments, the outputs of a localization network are usually image coordinates containing the object of interest. However, if a SDS-like architecture is used, the output of the network may also contain what is called an instance segmentation. If automated measurements the EAT layer are desired, either SDS networks may be used to segment out the EAT or the EAT predicted bounding boxes from SSD-like nets may be used to trigger an additional deep learning segmentation network.

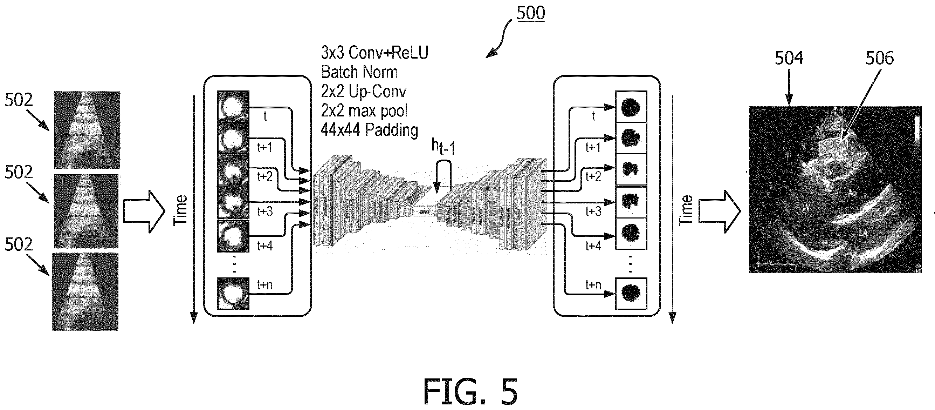

**FIG. 5** shows an example of a neural network 500 in accordance with the principles of the present disclosure. In the example shown in FIG. 5, the neural network 500 has an SDS-like architecture. The neural network 500 may receive a series of ultrasound images 502. The ultrasound images 502 may be acquired at different times and/or at different phases of the cardiac cycle. The neural network 500 may segment the EAT from the images as well as determine the cardiac phase during which the image was acquired. The output of the neural network 500 may be one or more images 504 where the EAT 506 has been segmented. Measurements of the EAT 506 may be acquired from the images 504. The measurements may be acquired by the neural network 500, another neural network and/or image processing technique, and/or a user. In some examples, EAT measurements (e.g., thickness across the long and short axes, total volume in the case of 3D images) for various phases of the cardiac cycle may be acquired and stored (e.g., systolic EAT PSAX, systolic EAT PLAX, systolic EAT mean, diastolic EAT PSAX, diastolic EAT PLAX, etc.).

Optionally, if patient's information is available, in some examples, a model that takes into account the measured EAT measurements and the patient information may be used to make a binary decision of whether patients need a follow-up appointment for CAD risk assessment. In some embodiments, the model may be included in neural network 410 and/or neural network 500. In some examples, the model may be a separate model that receives the outputs of one or more neural networks as inputs (e.g., the EAT measurements calculated by neural network 500). In some examples, the model may include a multivariate logistic regression. The multivariate logistic regression may be trained on current and/or retrospective datasets from clinical studies where patient clinical information and EAT measurements are both available. The dataset may include but is not limited to a list of clinical factors related to the patient (e.g., BMI, age, sex, smoke or not etc.) and/or clinical findings collected (e.g., laboratory findings including HDL cholesterol, triglyceride etc.) through current or previous studies. In some examples, the multivariate logistic regression may output a predictive score that is an indication of the likelihood the patient has CAD based on the combination of patients' medical history and EAT measurement evaluations. The predictive score may be used to provide the binary decision. For example, the decision to provide a report that the patient should have a follow-up may be made when the predictive score is equal to or above a threshold value.

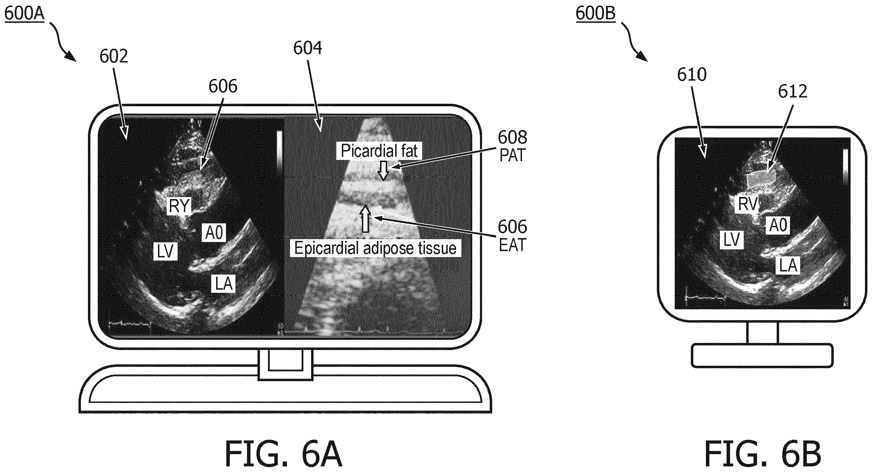

**FIG. 6A** is an example of a dual-display of ultrasound images in accordance with the principles of the present disclosure. In some examples, display 600A may be included in display 138. As discussed in reference to FIG. 1, in some examples, EAT processor 170 may cause the imaging system 100 to acquire images of the proper cardiac view and an enhanced visualization of the EAT in an interleaved acoustic sequences. As shown in FIG. 6A, user may see a split window with a side-by-side display of a conventional parasternal (e.g., PLAX, PSAX) view 602 and an enhanced view 604 of the EAT 606 of top part of the right ventricle. In the example shown in FIG. 6A, the PAT 608 may also be seen.

**FIG. 6B** is an example of a display of an ultrasound image in accordance with the principles of the present disclosure. In some examples, display 600B may be included in display 138. As discussed in reference to FIG. 1, the EAT processor 170 may identify the pixels and/or voxels that include the EAT. The identified pixels and/or voxels 612 may be overlaid onto the ultrasound image 610. In some embodiments, the overlaid pixels and/or voxels 612 may be a different color (e.g., green, red, yellow) or intensity (e.g., maximum intensity) than the pixels and/or voxels of the ultrasound image 610.

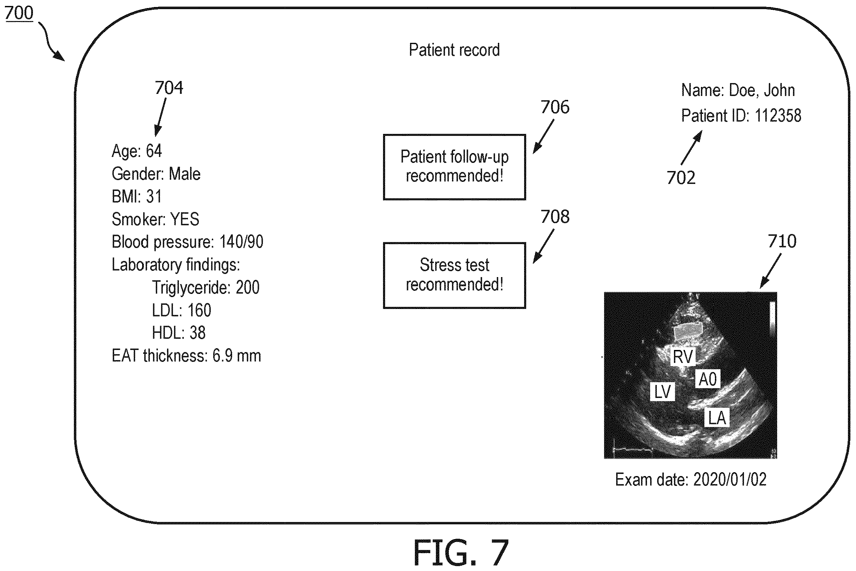

**FIG. 7** is an example of a patient dashboard 700 in accordance with the principles of the present disclosure. In some examples, the patient dashboard 700 may be provided on a display, such as display 138. The dashboard 700 may provide patient identifying information 702 such as name and/or identification number. The dashboard 700 may provide some or all of the patient's information 704 from their patient history (e.g., medical records). Patient information 704 may include age, gender, blood pressure. EAT measurements may be provided with the patient information 704. Optionally, in some examples, if any of the values of the patient information 704 fall outside a normal range (e.g., the value for EAT thickness, the value for HDL), that particular value may be flagged. The value may be flagged by providing the value in a different color, highlighting the value, placing a symbol (e.g., star) next to the value, and/or other flagging mechanism. Optionally, images, such as a cardiac ultrasound image 710 may be provided by the dashboard 700. The dashboard 700 may provide a report 706 if a follow-up is needed. Optionally, the dashboard 700 may provide a report 708 of a type of follow-up (e.g., stress test, angiography, CT, blood test). The reports 706, 708 may be based, at least in part, on the EAT measurements and the patient information as described herein, for example, using a regression model.

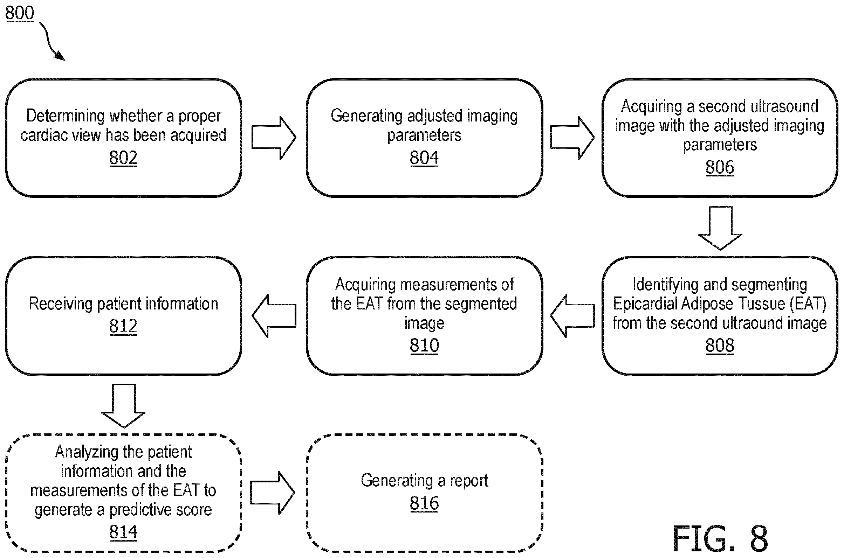

**FIG. 8** is a flow chart of a method 800 in accordance with the principles of the present disclosure. The method 800 may be performed by ultrasound imaging system 100 in some examples.

At block 802, "determining whether a proper cardiac view has been acquired" may be performed. The cardiac view may be searched for in an ultrasound image acquired by an ultrasound probe 112. The determining may be performed by a processor, such as EAT processor 170. As discussed, the proper cardiac view may be a standard view from an echo cardiology exam, such as the PSAX and PLAX view. In some examples, the determination may be based, at least in part, on detection and/or characterization of one or more anatomical cardiac landmarks included in the image.

When it is determined that the proper cardiac view has been acquired, at block 804, "generating adjusted imaging parameters" may be performed. The adjusted imaging parameters may be determined by the processor. Adjusting the imaging parameters may include increasing a center frequency, decreasing a focal depth, and/or increasing a gain. In some examples, the adjusted imaging parameters may be provided to a transmit controller, beamformer, probe, and/or other components of the ultrasound imaging system. At block 806, "acquiring a second ultrasound image with the adjusted imaging parameters" may be performed.

At block 808, "identifying and segmenting epicardial adipose tissue (EAT) from the second ultrasound image" may be performed. In some examples, this may be performed by the processor. The segmenting may be used to generate a segmented image.

At block 810, "acquiring measurements of the EAT from the segmented image" may be performed. In some examples, this may be performed by the processor. In some examples, the EAT measurements may include a thickness of the EAT along one or more axes.

Optionally, at block 812, "receiving patient information" and block 814 "analyzing the patient information and the measurements of the EAT to generate a predictive score" may be performed. In some examples, the patient information may be received from a local memory. Optionally, at block 816, "generating a report" may be performed. In some examples, the report may recommend a patient follow-up, based at least in part, on the predictive score. In some embodiments, blocks 812 and 814 may be performed by a processor not included with the imaging system. For example, a processor included with a workstation (e.g., a radiologist's workstation). The EAT measurements may be retrieved from an ultrasound imaging system (e.g., system 100) and patient information may be stored on the workstation or retrieved from a patient record database, cloud computing system, and/or other storage location.

Optionally, determining a phase of the cardiac cycle of the second ultrasound image may be performed. This may be performed before, after, or simultaneously with block 808.

In some embodiments, block 802, block 804, block 808, block 814, and/or block 816 may be performed by one or more neural networks. In some embodiments, one or more of the neural networks may have a simultaneous detection and segmentation (SDS) architecture. In some examples, prior to block 802, training the one or more neural networks with a training set may be performed. In some examples, the training set may include ultrasound images annotated with one or more anatomical cardiac landmarks.

The systems and methods disclosed herein may provide improved visualization and segmentation of EAT, which may provide more robust measurements of EAT. With a predictive score and follow-up commendation, the present disclosure may provide an end-to-end solution to CAD screening. The systems and methods disclosed herein may not only standardize EAT measurements, but may also guide the user in decision making on the need of a further in-depth follow-up appointment.

Although the examples described herein discuss processing of ultrasound image data, it is understood that the principles of the present disclosure are not limited to ultrasound and may be applied to image data from other modalities such as magnetic resonance imaging and computed tomography.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "C#", "Java", "Python", and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

## Claims

1. An ultrasound imaging system (100) comprising:
a processor (170) configured to:
analyze a first ultrasound image to determine whether a proper cardiac view has been acquired;
when the proper cardiac view has been acquired, adjust imaging parameters;
cause an ultrasound probe to acquire a second ultrasound image with the adjusted imaging parameters; and
identify and segment epicardial adipose tissue (EAT) from the second ultrasound image to generate a segmented image.

2. The ultrasound imaging system (100) of claim 1, wherein the processor (170) is further configured to acquire measurements of the EAT from the segmented image.

3. The ultrasound imaging system (100) of claim 2, wherein the processor (170) is further configured to receive patient information and generate a report based, at least in part, on the patient information and the measurements of the EAT.

4. The ultrasound imaging system (100) of claim 3, wherein the report is generated with a multivariate logistic regression model or wherein the report includes whether or not a patient requires a follow-up.

5. The ultrasound imaging system (100) of claim 1, wherein the processor (170) is further configured to output pixels (612) including the EAT and the pixels are overlaid on the second ultrasound image (610).

6. The ultrasound imaging system (100) of claim 1, wherein the processor (170) is further configured to cause the ultrasound probe (112) to acquire the second ultrasound image with the adjusted imaging parameters and a third ultrasound image with original imaging parameters in an interleaved manner, and optionally wherein the ultrasound imaging system (100) further comprises a display (138), wherein the second ultrasound image and the third ultrasound image are provided on the display simultaneously.

7. The ultrasound imaging system (100) of claim 1, wherein the processor (170) implements at least one neural network configured to determine when the proper cardiac view has been acquired.

8. A computer-implemented method (800) comprising:
determining (802) whether a proper cardiac view has been acquired in a first ultrasound image;
responsive to determining the proper cardiac view has been acquired, generating (804) adjusted imaging parameters;
acquiring (806) a second ultrasound image with the adjusted imaging parameters;
identifying and segmenting (808) epicardial adipose tissue (EAT) from the second ultrasound image to generate a segmented image; and
acquiring measurements (810) of the EAT from the segmented image.

9. The method (800) of claim 8, further comprising determining a phase of a cardiac cycle of the second ultrasound image.

10. The method (800) of claim 8, wherein the determining is based, at least in part, on detecting at least one anatomical cardiac landmark in the first ultrasound image.

11. The method (800) of claim 8, wherein generating the adjusted imaging parameters includes at least one of increasing a center frequency, decreasing a focal depth, or increasing a gain.

12. The method (800) of claim 8, wherein at least one of the determining, the generating, or the segmenting is performed by one or more neural networks.

13. The method (800) of claim 12, further comprising training the one or more neural networks with a training set, wherein the training set includes ultrasound images annotated with one or more anatomical cardiac landmarks or wherein at least one of the one or more neural networks has a simultaneous detection and segmentation (SDS) architecture.

14. The method (800) of claim 8, further comprising:
receiving (812) patient information; and
analyzing (814) the patient information and the measurements of the EAT to generate a predictive score; and optionally further comprising generating (816) a report, wherein the report recommends a patient follow-up when the predictive score, based at least in part, on the predictive score.

15. A non-transitory computer-readable medium may contain instructions, that when executed, causes an imaging system (100) to:
determine (802) whether a proper cardiac view has been acquired in a first ultrasound image;
responsive to determining the proper cardiac view has been acquired, generate (804) adjusted imaging parameters;
acquire (806) a second ultrasound image with the adjusted imaging parameters;
identify and segment (808) epicardial adipose tissue (EAT) from the second ultrasound image to generate a segmented image; and
acquire (810) measurements of the EAT from the segmented image; and
optionally wherein the instructions when executed, further cause the imaging system to:
acquire the second ultrasound image with the adjusted imaging parameters and a third ultrasound image with original imaging parameters in an interleaved manner; and
display the second ultrasound image and the third ultrasound image simultaneously.

## Patentansprüche

1. Ultraschallbildgebungssystem (100), umfassend:
einen Prozessor (170), der dazu konfiguriert ist:
ein erstes Ultraschallbild zu analysieren, um zu bestimmen, ob eine geeignete Herzansicht erfasst wurde.
wenn die geeignete Herzansicht erfasst wurde, die Bildgebungsparameter anzupassen;
zu veranlassen, dass eine Ultraschallsonde ein zweites Ultraschallbild mit den angepassten Bildgebungsparametern erfasst; und
epikardiales Fettgewebe (EAT) aus dem zweiten Ultraschallbild zu identifizieren und zu segmentieren, um ein segmentiertes Bild zu erzeugen.

2. Ultraschallbildgebungssystem (100) nach Anspruch 1, wobei der Prozessor (170) weiter dazu konfiguriert ist, Messungen des EAT aus dem segmentierten Bild zu erfassen.

3. Ultraschallbildgebungssystem (100) nach Anspruch 2, wobei der Prozessor (170) weiter dazu konfiguriert ist, Patienteninformationen zu empfangen und einen Bericht mindestens zum Teil basierend auf den Patienteninformationen und den Messungen des EAT zu erzeugen.

4. Ultraschallbildgebungssystem (100) nach Anspruch 3, wobei der Bericht mit einem multivariaten logistischen Regressionsmodell erzeugt wird, oder wobei der Bericht beinhaltet, ob ein Patient eine Nachuntersuchung benötigt oder nicht.

5. Ultraschallbildgebungssystem (100) nach Anspruch 1, wobei der Prozessor (170) weiter dazu konfiguriert ist, Pixel (612), einschließlich des EAT, auszugeben, und die Pixel auf dem zweiten Ultraschallbild (610) überlagert werden.

6. Ultraschallbildgebungssystem (100) nach Anspruch 1, wobei der Prozessor (170) weiter dazu konfiguriert ist, die Ultraschallsonde (112) zu veranlassen, das zweite Ultraschallbild mit den angepassten Bildgebungsparametern und ein drittes Ultraschallbild mit ursprünglichen Bildgebungsparametern in einer verschachtelten Weise zu erfassen, und wobei das Ultraschallbildgebungssystem (100) wahlweise weiter eine Anzeige (138) umfasst, wobei das zweite Ultraschallbild und das dritte Ultraschallbild gleichzeitig auf der Anzeige bereitgestellt werden.

7. Ultraschallbildgebungssystem (100) nach Anspruch 1, wobei der Prozessor (170) mindestens ein neuronales Netz implementiert, das dazu konfiguriert ist zu bestimmen, wenn die geeignete Herzansicht erfasst wurde.

8. Computerimplementiertes Verfahren (800), umfassend:
Bestimmen (802), ob in einem ersten Ultraschallbild eine geeignete Herzansicht erfasst wurde;
als Reaktion auf das Bestimmen, dass die geeignete Herzansicht erfasst wurde, Erzeugen (804) angepasster Bildgebungsparameter;
Erfassen (806) eines zweiten Ultraschallbildes mit den angepassten Bildgebungsparametern;
Identifizieren und Segmentieren (808) von epikardialem Fettgewebe (EAT) aus dem zweiten Ultraschallbild, um ein segmentiertes Bild zu erzeugen; und
Erfassen von Messungen (810) des EAT aus dem segmentierten Bild.

9. Verfahren (800) nach Anspruch 8, das weiter das Bestimmen einer Phase eines Herzzyklus des zweiten Ultraschallbildes umfasst.

10. Verfahren (800) nach Anspruch 8, wobei das Bestimmen mindestens zum Teil auf dem Erkennen mindestens eines anatomischen Herzmerkmals in dem ersten Ultraschallbild basiert.

11. Verfahren (800) nach Anspruch 8, wobei das Erzeugen der angepassten Bildgebungsparameter mindestens eines aus Erhöhen einer Mittenfrequenz, Verringern einer Brennweite oder Erhöhen einer Verstärkung beinhaltet.

12. Verfahren (800) nach Anspruch 8, wobei mindestens eines des Bestimmens, des Erzeugens oder des Segmentierens durch ein oder mehrere neuronale Netze durchgeführt wird.

13. Verfahren (800) nach Anspruch 12, das weiter das Trainieren des einen oder der mehreren neuronalen Netze mit einem Trainingssatz umfasst, wobei der Trainingssatz Ultraschallbilder beinhaltet, die mit einem oder mehreren anatomischen Herzmerkmalen vermerkt sind, oder wobei mindestens eines des einen oder der mehreren neuronalen Netze eine Architektur zur simultanen Erkennung und Segmentierung (SDS) aufweist.

14. Verfahren (800) nach Anspruch 8, weiter umfassend:
Empfangen (812) von Patienteninformationen; und
Analysieren (814) der Patienteninformationen und der EAT-Messungen, um einen Vorhersagewert zu erzeugen; und das wahlweise weiter Erzeugen (816) eines Berichts umfasst, wobei der Bericht eine Nachuntersuchung des Patienten empfiehlt, wenn der Vorhersagewert mindestens zum Teil auf dem Vorhersagewert basiert.

15. Nichtflüchtiges computerlesbares Medium, das Anweisungen beinhalten kann, die bei Ausführung ein Ultraschallbildgebungssystem (100) dazu veranlassen:
zu bestimmen (802), ob in einem ersten Ultraschallbild eine geeignete Herzansicht erfasst wurde;
als Reaktion auf das Bestimmen, dass die geeignete Herzansicht erfasst wurde, angepasste Bildgebungsparameter zu erzeugen (804);
ein zweites Ultraschallbild mit den angepassten Bildgebungsparametern zu erfassen (806);
epikardiales Fettgewebe (EAT) aus dem zweiten Ultraschallbild zu identifizieren und zu segmentieren (808), um ein segmentiertes Bild zu erzeugen; und
Messungen des EAT aus dem segmentierten Bild zu erfassen (810); und
wobei die Anweisungen, wenn sie ausgeführt werden, wahlweise das Bildgebungssystem weiter dazu veranlassen:
das zweite Ultraschallbild mit den angepassten Bildgebungsparametern und ein drittes Ultraschallbild mit ursprünglichen Bildgebungsparametern in verschachtelter Weise zu erfassen; und
das zweite Ultraschallbild und das dritte Ultraschallbild gleichzeitig anzuzeigen.

## Revendications

1. Système d'imagerie ultrasonore (100) comprenant :
un processeur (170) configuré pour :
analyser une première image ultrasonore pour déterminer si une vue cardiaque appropriée a été acquise ;
lorsque la vue cardiaque appropriée a été acquise, ajuster les paramètres d'imagerie ;
amener une sonde ultrasonore à acquérir une deuxième image ultrasonore avec les paramètres d'imagerie ajustés ; et
identifier et segmenter le tissu adipeux épicardique (EAT) à partir de la deuxième image ultrasonore pour générer une image segmentée.

2. Système d'imagerie ultrasonore (100) selon la revendication 1, dans lequel le processeur (170) est en outre configuré pour acquérir des mesures de l'EAT à partir de l'image segmentée.

3. Système d'imagerie ultrasonore (100) selon la revendication 2, dans lequel le processeur (170) est en outre configuré pour recevoir des informations de patient et générer un rapport basé, au moins en partie, sur les informations de patient et les mesures de l'EAT.

4. Système d'imagerie ultrasonore (100) selon la revendication 3, dans lequel le rapport est généré avec un modèle de régression logistique multivariée ou dans lequel le rapport inclut si un patient nécessite, ou non, un suivi.

5. Système d'imagerie ultrasonore (100) selon la revendication 1, dans lequel le processeur (170) est en outre configuré pour délivrer des pixels (612) incluant l'EAT et les pixels sont superposés sur la deuxième image ultrasonore (610).

6. Système d'imagerie ultrasonore (100) selon la revendication 1, dans lequel le processeur (170) est en outre configuré pour amener la sonde ultrasonore (112) à acquérir la deuxième image ultrasonore avec les paramètres d'imagerie ajustés et une troisième image ultrasonore avec des paramètres d'imagerie d'origine d'une manière entrelacée et, facultativement, dans lequel le système d'imagerie ultrasonore (100) comprend en outre un affichage (138), dans lequel la deuxième image ultrasonore et la troisième image ultrasonore sont fournies simultanément sur l'affichage.

7. Système d'imagerie ultrasonore (100) selon la revendication 1, dans lequel le processeur (170) met en oeuvre au moins un réseau neuronal configuré pour déterminer le moment auquel la vue cardiaque appropriée a été acquise.

8. Procédé mis en oeuvre par ordinateur (800) comprenant :
la détermination (802) si une vue cardiaque appropriée a été, ou non, acquise dans une première image ultrasonore ;
à la suite de la détermination que la vue cardiaque appropriée a été acquise, la génération (804) de paramètres d'imagerie ajustés ;
l'acquisition (806) d'une deuxième image ultrasonore avec les paramètres d'imagerie ajustés ;
l'identification et la segmentation (808) du tissu adipeux épicardique (EAT) à partir de la deuxième image ultrasonore pour générer une image segmentée ; et
l'acquisition de mesures (810) de l'EAT à partir de l'image segmentée.

9. Procédé (800) selon la revendication 8, comprenant en outre la détermination d'une phase d'un cycle cardiaque de la deuxième image ultrasonore.

10. Procédé (800) selon la revendication 8, dans lequel la détermination est basée, au moins en partie, sur la détection d'au moins un repère cardiaque anatomique dans la première image ultrasonore.

11. Procédé (800) selon la revendication 8, dans lequel la génération des paramètres d'imagerie ajustés inclut au moins une de l'augmentation d'une fréquence centrale, de la diminution d'une profondeur focale et de l'augmentation d'un gain.

12. Procédé (800) selon la revendication 8, dans lequel au moins une de la détermination, de la génération et de la segmentation est effectuée par un ou plusieurs réseaux neuronaux.

13. Procédé (800) selon la revendication 12, comprenant en outre l'entraînement du ou des réseaux neuronaux avec un ensemble d'entraînement, dans lequel l'ensemble d'entraînement inclut des images ultrasonores annotées avec un ou plusieurs repères cardiaques anatomiques ou dans lequel au moins un des un ou plusieurs réseaux neuronaux présente une architecture de détection et de segmentation simultanées (SDS).

14. Procédé (800) selon la revendication 8, comprenant en outre :
la réception (812) d'informations de patient ; et
l'analyse (814) des informations de patient et des mesures de l'EAT pour générer un score prédictif; et, facultativement, comprenant en outre la génération (816) d'un rapport, dans lequel le rapport recommande un suivi du patient sur la base, au moins en partie, du score prédictif.

15. Support non transitoire lisible par ordinateur qui peut contenir des instructions qui, lorsqu'elles sont exécutées, amènent un système d'imagerie (100) à :
déterminer (802) si une vue cardiaque appropriée a été, ou non, acquise dans une première image ultrasonore ;
à la suite de la détermination que la vue cardiaque appropriée a été acquise, générer (804) des paramètres d'imagerie ajustés ;
acquérir (806) une deuxième image ultrasonore avec les paramètres d'imagerie ajustés ;
identifier et segmenter (808) le tissu adipeux épicardique (EAT) à partir de la deuxième image ultrasonore pour générer une image segmentée ; et
acquérir (810) des mesures de l'EAT à partir de l'image segmentée ; et
facultativement, dans lequel les instructions, lorsqu'elles sont exécutées, amènent en outre le système d'imagerie à :
acquérir la deuxième image ultrasonore avec les paramètres d'imagerie ajustés et une troisième image ultrasonore avec les paramètres d'imagerie d'origine de manière entrelacée ; et
afficher simultanément la deuxième image ultrasonore et la troisième image ultrasonore.
